# EUROPEAN PATENT SPECIFICATION

(11) **EP 1 814 529 B1**
(45) Date of publication and mention of the grant of the patent: **20.07.2016**
(21) Application number: 05801533.0
(22) Date of filing: 28.10.2005
(51) Int. Cl.: A61K 9/28, A61K 9/30, A61K 31/519, A61P 25/20

(54) **DOSAGE FORM TIME-LAGGED OF DRUGS FOR THE THERAPY OF INSOMNIA**
ZEITVERZÖGERTE DOSIERFORM VON ARZNEIMITTELN ZUR BEHANDLUNG VON SCHLAFLOSIGKEIT
FORME POSOLOGIQUE POUR MEDICAMENT À TEMPS DE LATENCE CONTRE L'INSOMNIE

(30) Priority: 28.10.2004 GB 0423964
(43) Date of publication of application: 08.08.2007
(60) Divisional application: 10186051.8
(73) Proprietor: Jagotec AG, 4132 Mutenz (CH)
(72) Inventor: VERGNAULT, Guy, F-68680 Kembs Loechle (FR); GRENIER, Pascal, 68510 Kappelen (FR)
(74) Representative: Crump, Julian Richard John
(86) International application number: PCT/EP2005/011568
(87) International publication number: WO 2006/045618

(56) References cited:
- EP-A- 0 605 174
- EP-A- 1 064 937
- WO-A-2004/045589
- US-B1- 6 183 780
- US-B1- 6 485 746
- 'AqualonTM Ethylcellulose' SIGNETCHEM, [Online] 2008, Hercules Inc. Retrieved from the Internet: <URL:http://www.signetchem.com/downloads/da tasheets/Ashland-Aqualon/PDS-Aqualon-EC-spe cifications.pdf>
- Maryam Maghsoodi ET AL: "Polymer Percolation Threshold in Multi-Component HPMC Matrices Tablets", Advanced Pharmaceutical Bulletin, 1 January 2011 (2011-01-01), pages 27-33, XP055223157, DOI: 10.5681/apb.2011.004 Retrieved from the Internet: URL:http://www.ncbi.nlm.nih.gov/pmc/articl es/PMC3850001/pdf/apb-1-27.pdf [retrieved on 2015-10-23]
- TAMARA GONÇALVES-ARAÚJO ET AL: 'Polymer Percolation Threshold in HPMC Extended Release Formulation of Carbamazepine and Verapamil HCl' AAPS PHARMSCITECH vol. 11, no. 2, 30 March 2010, pages 558 - 562, XP055225388 DOI: 10.1208/s12249-010-9408-x

## Description

The present invention is concerned with methods and compositions for treating insomnia in human subjects.

Many pathologies or conditions are related to abnormalities within diurnal rhythms. Insomnia is such a condition. However, whereas insomnia is a very prevalent condition it is generally considered among physicians that many people are amenable to pharmacologic intervention to help ameliorate their problems.

When assessing the symptoms of insomnia, physicians have found that they fall generally within the categories of i) latency to sleep, ii) duration of sleep, iii) disturbed patterns of sleep, i.e. frequent nocturnal wakening events, and iv) residual hangover effects upon awakening such as drowsiness and impairment of cognitive and motor functions.

Early treatments for insomnia commonly employed central nervous system (CNS) depressants such as barbiturates. These compounds typically have long half lives and have a well-known spectrum of side effects, including lethargy, confusion, depression and next day hangover effects. In addition, chronic use has been associated with a high potential for addiction involving both physical and psychological dependence.

Treatments moved away from barbiturates and other CNS depressants toward the benzodiazepine class of sedative-hypnotic agents. This class of compounds produces a calming effect that results in a sleep-like state in humans and animals, with a greater safety margin than prior hypnotics. However, many benzodiazepines possess side effects that limit their usefulness in certain patient populations. These problems include synergy with other CNS depressants (especially alcohol), the development of tolerance upon repeat dosing, rebound insomnia following discontinuation of dosing, hangover effects the next day and impairment of psychomotor performance and memory.

More recent treatments for insomnia have used non-benzodiazepine compounds. Ambien (zolpidem), Sonata (zaleplon) are examples of approved drug products. Zaleplon, also known as N-[3-(3-cyanopyrazole[1,5-a] pyrimidin-7-yl)phenyl]-N-ethylacetamide, is a pyrazolopyrimidine hypnotic that binds selectively to the benzodiazepine type I site on the GABA-A (γ-aminobutyric acid, type A) receptor complex. Other non-benzodiazepine compounds useful in the treatment of insomnia are known in the literature.

What is clear, however, is that there is still hesitance on the part of patients and physicians with regard to the use of sedatives and other CNS active agents in a chronic setting. Despite huge improvements in available drug substances, pharmacological intervention cannot rely solely on the properties inherent to these drug substances alone. The way in which such drug substances are formulated will largely influence their efficacy, side-effect profiles, and ultimately the acceptance by both patients and physicians alike. Given the huge growth potential of the insomnia market, in addition to the current therapies and the search for improved drug substances, not surprisingly many companies are turning their attention to the development of improved therapeutics for existing drug substances.

Certain sedatives are commonly available or are in development in the form of immediate release dosage forms. As is well known in the art, immediate release dosage forms provide a burst of drug substance shortly after ingestion, to induce rapid onset of sleep. Whereas such dosage forms address the latency to sleep problem, unless the drug substance has a long half life, in order to maintain effective blood plasma concentration levels over an extended period of time, patients experiencing short sleep duration or frequent nocturnal awakening events will need to take further dosage forms during the night to maintain sleep.

Modified release dosage forms produce an initial burst of drug substance to induce rapid onset of sleep, and continue to release drug substance in a controlled manner to maintain effective plasma concentrations over an extended period of time to improve sleep maintenance. A potential disadvantage of this approach is the time to clearance of the active substance from a patient's system. Drug substance still present at effective levels can cause hangover effects upon wakening.

A particular modified release dosage form is described in US patent 6,485,746. In this patent there is described a formulation of a sedative-hypnotic compound that provides a pulsatile release profile in vivo whereby upon administration the drug substance is released rapidly to provide a maximum plasma concentration within 0.1 to 2 hours following administration. Thereafter, plasma concentration passes through a minimum at about 2 to 4 hours post administration, before a second pulse delivers a second maximum plasma concentration at about 3 to 5 hours. Finally, after 8 hours there remains a plasma concentration that represents no more than 20% of the plasma concentration of the second maximum.

Existing formulations and those in development are only concerned with improving the quality of sleep and the prevention of hangover effects. None, as far as applicant is aware, address the problems that sedatives can create to a patient's pre-sleep routine. The rapid onset of drowsiness, and the concomitant disruption of pre-sleep activities such as reading and watching TV, may result in increased hesitance of physicians to prescribe a drug, and poorer patient compliance.

Sedation affecting pre-sleep routines is an unpleasant aspect of insomnia medications, made more so when one considers that a high proportion of insomnia sufferers do not complain of problems falling asleep, but are only afflicted by short sleep duration and frequent nocturnal awakening events. Furthermore, there is evidence suggesting a significant placebo effect associated with therapies intended to initiate a rapid onset of sleep.

EP 0605174 A1 describes a sustained release pharmaceutical preparation comprising a water-soluble drug core, surround by a hydratable diffusion barrier; US 6183780 B1 describes an oral delayed, immediate release formulation which comprises a compressed core and a coating; and WO 2004/044589 A1 describes a dosage form comprising a short-acting hypnotic for the treatment of sleep disorders.

Despite the increased activity in the development of therapeutics in this area, there remains a need to offer patients a dosage form that can be taken before bedtime that not only provides extended sleep duration and reduces or eliminates nocturnal awakening events, but which leaves patients free to go about pre-sleep activities un-sedated.

Accordingly, the invention provides in a first aspect a dosage form for use in a method of treating insomnia in a patient in need thereof, comprising administering a dosage form containing a drug substance useful in treating insomnia, the dosage form being adapted to release said drug substance after a lag time during which no, or substantially no, drug substance is released, the lag time being about from 1 to 4 hours after administration of the dosage form.

In contrast to certain dosage forms described in the prior art, the dosage form used in the method of the present invention is adapted to release the active drug substance in a time-dependent manner, i.e. after a pre-determined lag time. No extrinsic changes in the environment, such as a change in pH or temperature, are required in order to prompt release of the drug substance from the dosage form after said pre-determined lag time.

The lag time is from 1 to 4 hours, more particularly from 1 to 2 or from 2 to 3 hours.

The pH of the gastric tract can differ markedly depending on whether a patient is in a fed or fasted state. Accordingly, to achieve a reliable pre-determined lag time, the release of said drug substance from the dosage form is preferably pH-independent.

In a preferred composition, during the pendency of lag time any drug substance that is released is in such small amounts that effective blood plasma levels of the drug substance are not reached. Preferably, drug substance release is less than 10% by weight, more particularly less than 5%, still more particularly less than 2%, still more particularly less than 1%.

Following the expiry of the lag time, the drug substance is released from the dosage form. The drug substance may for example be released rapidly (immediate release) or may be released slowly over a period of time (modified release). Preferably the drug substance may be released in a non-pulsatile manner. Thus, the drug substance may be released from the dosage form at a steady or continuous rate.

Lag time can be measured *in vitro* using dissolution methods and apparatus generally known in the art. The United States Pharmacopoeia describes several such methods.

In a particular embodiment of the present invention, there is provided a dosage form for use in a method of treating insomnia in a patient in need thereof comprising administering a dosage form containing a drug substance useful in treating insomnia, the dosage form being adapted to release said drug substance after a lag time during which no, or substantially no, drug substance is released, the lag time being about from 1 to 4 hours after administration of the dosage form, which dosage form is adapted to obtain a controlled release of said drug substance in vitro when measured by the USP Paddle Method (type II apparatus) at 100 rpm, in 1000ml of an aqueous medium such that during said lag time, not more than 10% of drug substance is released.

In another particular embodiment of the present invention, there is provided a dosage form for use in a method of treating insomnia in a patient in need thereof comprising administering a dosage form containing a drug substance useful in treating insomnia, the dosage form being adapted to release said drug substance after a lag time during which no, or substantially no, drug substance is released, the lag time being about from 1 to 4 hours after administration of the dosage form, which dosage form is adapted to obtain a controlled release of said drug substance in vitro when measured by the USP Paddle Method (type II apparatus) at 100 rpm at 37°C in 1000 ml of (a) 0.1 M HCl and phosphate buffer (pH 6.8) or (b) 0.02% sodium lauryl sulphate in 500ml distilled water or (c) purified water, such that during said lag time not more than 10% of drug substance is released.

More particularly, in a dosage form for use in a method according to the present invention a dosage form is adapted to obtain a controlled release of said drug substance in vitro when measured by the USP Paddle Method (type II apparatus) at 100 rpm, in 1000ml of an aqueous medium such that during said lag time not more than 10% of drug substance is released, at least about 25 to 60% is released within 5 hours, and at least about 80% is released after 7 hours.

Still more particularly in a method according to the present invention a dosage form is adapted to obtain a controlled release of said drug substance in vitro when measured by the USP Paddle Method (type II apparatus) at 100 rpm at 37°C in 1000 ml of (a) 0.1M HCl and phosphate buffer (pH 6.8) or (b) 0.02% sodium lauryl sulphate in 500ml distilled water or (c) purified water, in an aqueous medium such that during said lag time not more than 10% of drug substance is released, at least about 25 to 60% is released within 5 hours, and at least about 80% is released after 7 hours.

The present invention provides a dosage form as claimed in claim 1. The invention further provides a dosage form useful in the above methods. Preferably, the dosage form is provided as a unit (single-component) dose.

Looked at from the perspective of both commercial and development products for the treatment of insomnia that work by delivering an immediate pulse of drug substance to combat latency to sleep problems, the present invention whereby the method of administration involves a lag time is counter-intuitive, and has certain advantages that are not shared with existing therapies. In this regard, we have already mentioned the benefits to a patient that is free to go about its pre-sleep activities without feeling sedated.

Although, the dosage form in accordance with the invention delivers the drug substance after a lag time, given the significant placebo effect referred to above it may be useful for treating or addressing sleep latency as well as wakening events.

Other advantages become apparent having regard to the biological processes associated with the sleep. The so-called "homeostatic process" is believed to be a primary driving force in creating in patients the need for sleep. For an individual having a bed time of around 11 p.m., this drive weakens in the early morning hours, e.g. around 3 a.m., and is further exacerbated by a circadian alert pulse around 5 a.m. that is believed to be an additional driver to wakefulness for patients. A lag time before drug release can ensure that peak plasma concentrations are reached several hours into the sleep cycle when nocturnal awakening events are likely to occur. By coinciding drug release and therefore maximum plasma concentrations with these processes occurring in the early morning hours, it may be possible to use lower doses of drug substances than would otherwise be needed using conventional sustained release dosage forms that must contain a significant amount of drug sub stance to provide the initial drug burst to arrest sleep latency problems.

Still further, many drug substances are metabolized by cytochrome CYP450 isoform 3A4, and this enzyme is present in relatively high concentrations in higher regions of the gastro-intestinal (GI) tract. A dosage form exhibiting a lag time may pass further down the GI tract before delivering drug substance in a region of lower CYP P450 activity, thereby potentially increasing the efficacy of the released drug substance. The front-line sedative hypnotic - zaleplon - is such a drug substance that is metabolized by CYP P450.

A dosage form in accordance with the present invention can deliver a drug substance such that a peak plasma concentration occurs around 3 a.m. in the morning (that is, around 4-5 hours after administration). Furthermore, using commonly available sustained release excipients (as will be further described herein below), drug substance plasma concentrations can be maintained at effective levels through 3 a.m. to coincide with the weakening homeostatic process and through 5 a.m. to coincide with a circadian alert pulse mentioned above.

Certain dosage forms described in the art are intended to achieve an extended sleep period of 8 hours. However, it is not always advantageous to deliver such an extended sleep pattern. Whereas, this might be a perfectly reasonable sleep pattern for people having less busy schedules, there are many individuals, e.g. travelers that need only to sleep for a short number of hours, e.g. 5 to 6 hours, before having to waken refreshed and alert. For such patients, it may not be considered advantageous to suppress the circadian alert pulse. The dosage forms useful in the method of the present invention are able to release a drug substance after a lag time in order to provide effective plasma concentrations of drug substance in order to coincide with the weakening homeostatic drive, and then permit the plasma levels to decay in a controllable manner to ensure a plasma levels are below effective levels between about 6 to 8 hours after administration, thereby avoiding or reducing the so-called "hangover effect".

The ability to avoid hangover effects, even after a relatively short sleep duration., e.g. of the order of 5 to 6 hours, may be more easily achieved by employing sedatives with short half lives, In general, a short-acting sedative is a compound that has a detectable sedative effect in any standard assay, with a mean plasma half-life of the compound of less than 2 hours. Of particular note in this regard is zaleplon, which has a half life of 1 hour.

The use of a short acting sedative in combination with the targeted dosing afforded by the dosage forms described herein, provides patients with the possibility of having relatively short sleep intervals and still wake up without experiencing hangover effects, or reduced hangover effects, and such a use forms a particularly preferred aspect of the present invention.

Drug substance for use in the present invention is zaleplon which is known to be useful for treating insomnia.

The amount of drug substance that may be employed will depend upon the type of drug substance, the type and severity of the condition to be treated, and the patient's medical history, age and weight. However, generally speaking drug substances may be administered in amounts to achieve a dose of from about 5 to 50 mg per day, more particularly 10 to 50 mg per day.

In the case of zaleplon, a unit dosage form for use in the method according to the invention may contain from 5 mg to 50 mg of drug substance, more particularly 5 mg to 25 mg of drug substance.

Dosage forms for the administration of a drug substance to improve sleep patterns in patients suffering with insomnia may take a variety of forms that are capable of presenting the drug substance in bioavailable form in effective amounts.

Dosage forms useful in the present invention contain one or more drug substances and a release controlling agent.

The release controlling agent may be in a matrix in which the drug substance is dissolved or dispersed. Alternatively, the release controlling agent may be in a layer or coating surrounding a drug substance-containing matrix. When the release controlling agent is in the layer or coating, the matrix may also contain a release controlling agent, or it may be adapted for immediate release of the drug substance.

By selecting appropriate matrix and/or coating materials one is able not only to accurately control the lag time, one is also to ensure that all, or substantially all, of the drug substance upon expiry of the lag time is released at a desired rate to achieve extend sleep patterns and eliminate or reduce nocturnal awakening events.

When selecting coating materials, it is preferred not to employ materials that are swellable or gellable. Typical of such materials are cellulose ethers or cellulosic derivatives such as hydroxyalkyl celluloses, e.g. hydroxypropylmethyl cellulose, or carboxyalkylcelluloses and the like. Such materials tend to form gels which exert a release-controlling effect by forming an erodible barrier through which drug substance may diffuse. Such materials tend to give unreliable lag times and should be avoided in amounts that exert a release-controlling effect. Their release-controlling properties are usually evident when they are employed in amounts of about 10% or greater. Therefore, if any of the aforementioned materials are employed as coating materials they should only be used in small amounts, e.g. less than 10%, more particularly less than 5%, still more particularly less than 1%.

The release controlling agent may comprise water-insoluble or poorly water soluble hydrophobic materials, such as waxy and insoluble excipients, that act by permitting ingress of aqueous physiological media through faults and channels in the bulk materials.

Release controlling agents may include hydrophilic and/or hydrophobic materials, such as gums, natural and synthetic waxes such as beeswax, glycowax, castor wax and carnauba wax, shellac, and mineral and vegetable oils such as hydrogenated castor oil, hydrogenated vegetable oil, polyalkylene glycols, long chain (e.g. 8 to 50 carbon atoms) substituted or unsubstituted hydrocarbon such as fatty acids and fatty alcohols, or glyceryl esters of fatty acids.

Release controlling agents may be present in the dosage form in amounts depending on the desired release profile. Such agents may be present in amounts of 1 to 99% by weight of the dosage form.

In addition to the above ingredients, a dosage form may also contain other excipients commonly employed in oral dosage forms such as diluents, lubricants, binders such as alkyl celluloses such as ethyl cellulose, granulating aids, colorants, flavorants and glidants. Examples of such ingredients include microcrystalline cellulose or calcium phosphate dibasic, calcium phosphate dihydrate, calcium sulfate dihydrate, cellulose derivatives, dextrose, lactose, anhydrous lactose, spray-dried lactose, lactose monohydrate, mannitol, starches, sorbitol and sucrose.

These excipients may be present in varying amounts consistent with obtaining a suitable oral dosage form. Excipients may be present in amounts of 1 to 99% by weight.

When a dosage form is intended to provide an immediate burst of drug substance after the lag time, the matrix may contain excipients commonly used in immediate release dosage forms.

A matrix adapted for an immediate burst of drug substance upon expiry of the lag time may comprise a surface-active agent such as sodium lauryl sulfate, sodium monoglycerate, sorbitan monooleate, polyoxyethylene sorbitan monooleate, glyceryl monostearate, glyceryl monooleate, glyceryl monobutyrate, any one of the Pluronic line of surface-active polymers, or any other suitable material with surface active properties or any combination of the above.

Surface active materials may be present in the dosage form in amounts of 0.5 to 10% by weight.

Other ingredients commonly employed in immediate release formulations include, but are not limited to, microcrystalline cellulose (such as A vicel), corn starch, pregelatinized starch (such as Starch 1500 or National 1551), potato starch, sodium carboxymethylated starch, sodium carboxymethylated cellulose, hydroxypropylmethyl cellulose, hydroxypropylcellulose, hydroxyethylcellulose, and ethylcellulose. In addition, binder materials such as gums (e.g., guar gum) natural binders and derivatives such as alginates, chitosan, gelatin and gelatin derivatives, are also useful. Synthetic polymers such as polyvinylpyrrolidone (PVP), acrylic acid derivatives (Eudragit, Carbopol, etc.) and polyethylene glycol (PEG) are also useful as binders and matrix formers. It may also be desirable to incorporate a disintegrant into an immediate release matrix in order to facilitate dissolution of the drug substance. For this purpose, any suitable tablet disintegrant can be utilized here, such as cross-linked sodium carboxymethylcellulose (Ac-Di-Sol), cross-linked sodium carboxymethyl starch (Explotab, Primojel), cross-linked PVP (Plasdone XL) or any other material possessing tablet disintegrant properties.

These ingredients may be present in the dosage form in amounts of 1 to 99% by weight.

As will be immediately apparent to the skilled person, a wide variety of release profiles can be obtained having regard to the nature and composition of the core matrix. In a particularly embodiment, the core may be of a multi-layered configuration, having both a release controlling layer and a layer for immediate release. In such an embodiment, it is preferred if the layers are rendered distinct each from the other. This may be achieved by one layer containing a colourant or a material that is opaque to x-rays, and the other not. The reason for this will become apparent from the description continued below.

Dosage forms described above may be over-coated with a pharmaceutically acceptable film-coating, for aesthetic purposes (e.g. including a colourant), for stability purposes (e.g., coated with a moisture barrier), for taste-masking purposes, or for the purpose of protecting unstable drug substances from aggressive media, e.g. enteric-coatings.

Dosage forms described above may be prepared according to any of the techniques known in the art. Matrices may be formed by mixing release controlling agent, drug substance and any suitable tabletting excipients, including any of those materials referred to above, and coated using techniques in the art.

For example, coatings may be formed by compression using any of the known press coaters. Alternatively, dosage forms may be prepared by granulation and agglomeration techniques, or built up using spray drying techniques, followed by drying.

Coating thickness can be controlled precisely by employing any of the aforementioned techniques. The skilled person can select the coating thickness as a means to obtain a desired lag time, and/or the desired rate at which drug substance is released after the lag time.

For reasons of patient compliance, preferably the dosage form should be as small as possible and the coating should have the minimum thickness possible consistent with achieving the desired lag time. By the judicious selection of the coating materials, one is able to produce a coating that is relative recalcitrant to the ingress of moisture and so long lag times can be achieved with relatively thin coatings.

Dosage forms may take any suitable form, including capsules, tablets and pellets. Such dosage forms may be intended for administration by any known means, including oral, buccal and sublingual. Preferably, the dosage form is adapted for oral delivery intended for ingestion.

A particularly preferred dosage form is provided in the form of a press-coated tablet. The tablet comprises a core containing a drug substance, and a coating surrounding said core, the core being applied by press-coating coating material around a preformed core. The coating may contain any of the release-controlling agents mentioned above.

The coating comprises one or more water insoluble or poorly soluble hydrophobic excipients selected from fatty acids or their esters or salts; long chain fatty alcohols; polyoxyethylene alkyl ethers; polyoxyethylene stearates; sugar esters; lauroyl rnacrogol-32 glyceryl, stearoyl macrogol-32 glyceryl, waxy substances, calcium phosphate salts and combinations thereof.

Other excipients that provide a hydrophobic quality to coatings may be selected from any waxy substance known for use as tablet excipients. Preferably they have a HLB value of less than 5, and more preferably about 2. Suitable hydrophobic agents include waxy substances such as carnauba wax, paraffin, microcrystalline wax, beeswax, cetyl ester wax and the like; or non-fatty hydrophobic substances such as calcium phosphate salts, e.g. dibasic calcium phosphate.

Coatings comprising the aforementioned materials provide for a lag time by acting as a barrier to the ingress of a physiological medium. Once the medium crosses the coating and enters the matrix causing the matrix to expand, for example by swelling, gelling or effervescing, the coating is broken open exposing the core matrix, thereby permitting release of drug substance from the matrix. In this way, the coating exerts no, or substantially no, influence over the release rate after expiry of the lag time.

Preferably coating ingredients are calcium phosphate salts, glyceryl behenate, and polyvinyl pyrollidone, or mixtures thereof, and one or more adjuvants, diluents, lubricants or fillers.

Preferred components in the coating are as follows, with generally suitable percentage amounts expressed as percentage weight of the coating.

Polyvinyl pyrollidone (Povidone) is preferably present in amounts of about 1 to 25% by weight or the coating, more particularly 4 to 12%, e.g. 6 to 8%.

Glyceryl behenate is an ester of glycerol and behenic acid (a C22 fatty acid). Glyceryl behenate may be present as its mono-, di-, or tri-ester form, or a mixture thereof. Preferably it has an HLB value of less than 5, more preferably approximately 2. It may be present in amounts of about 5 to 85% by weight of the coating, more particularly from 10 to 70% by weight, and in certain preferred embodiments from 30 to 50%.

Calcium phosphate salt may be the dibasic calcium phosphate dihydrate and may be present in an amount of about 10 to 90% by weight of the coating, preferably 20 to 80%, e.g. 40 to 75%.

The coating may contain other excipients commonly used in forming solid oral dosage forms, such as are described above.

As already stated above, the coating thickness surrounding the core will influence the lag time, and can also affect the rate of drug release thereafter depending on the nature of the coating materials selected. The applicant has found that press-coating provides a particularly effective means of controlling coating thickness, and therefore controlling the lag time.

Press-coating is particularly advantageous as one can control coat weight, diameter of die and size of core to achieve a precisely defined minimum coating thickness at points on the dosage form. Ingress of a physiological medium across the coating at these points will determine the time period for the medium to reach the core and hydrate it, and the lag time is controlled in this manner.

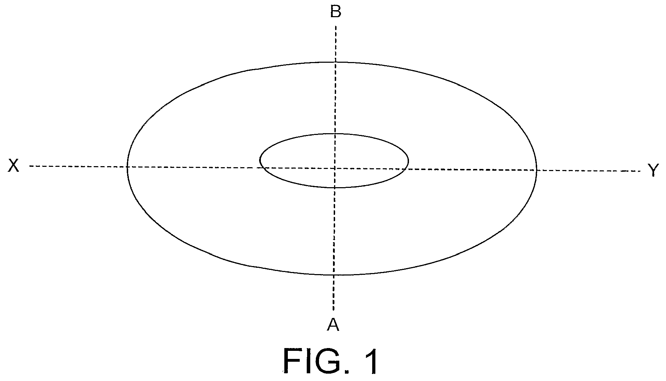

With reference to Figure 1 below, the thickness of the coating along and about the axis of the direction of movement of a press-coater punch (the "(A-B)" axis) is determined by the amount of coating material added to the die and the compaction force applied to form of a dosage form. On the other hand, the thickness of the coating along and about the "(X-Y)" axis is determined by the size of the core, its position within the die and the diameter of the die in the press-coater. It will be apparent to the skilled person that even though figure 1 only shows a 2-dimensional representation of a dosage form, there is a plurality of axes (X-Y) orthogonal to the "A-B" axis, which extend radially from the centre of the dosage form to its circumference, and when the reference is made to the thickness of the coating about an axis X-Y, reference is being made the thickness about any or all of these axes.

Given that one can manipulate the thickness of the coating around or about the axis (A-B) to ensure it is thicker than the coating about the axis (X-Y), ingress of moisture at X-Y will influence the lag time. Accordingly, the formulator has some latitude in selecting the thickness of the coating along A-B. It should not be so thick as to render the dosage form too large and therefore difficult to swallow, yet on the other hand it should not be so thin that the coating is render weak and liable to crack under the slightest mechanical stress.

In a preferred embodiment, a dosage form comprises a press-coated tablet comprising a core and a coating surrounding the core, the coating having thickness about the axis X-Y such that upon immersion in an aqueous medium as herein above described there will be less than 10% release of drug substance, more particularly less than 5%, still more particularly less than 2%, most particularly less than 1% during a lag time as defined herein above.

The thickness of the coating about the axis X-Y may be about 2 to 2.6mm.

The dosage form is formed by compression coating methods as will be described in more detail herein below. Compression coated dosage forms are generally formed by placing a portion of a powdered coating material in a die and tamping the powder into a compact form using a punch. A core is then deposited onto the compacted coating material before the remainder of the coating material is introduced into the die and compression forces are applied to form the coated dosage form. To ensure that the core is placed on the tamped coating material and to ensure its correct geometry relative to the coating in the final tablet form, it is preferable to employ means for positioning the core in relation to the coating material in a die. Typically such means may be provided by a pin punch. A pin punch is a punch that has a convex surface that contacts the coating material to leave a small depression or hollow in the tamped coating material. Thus, when the core is placed into the die on the tamped material, it sits in the depression or hollow and its correct geometry is assured in the final tablet form.

As a result of this process, different areas of the formed tablet may experience different compaction forces, and therefore the coating may vary in density or porosity at different points. For example, the top portion of the coating along axis A-B (in the direction of the movement of the punch) is generally more compact compared with the bottom portion along the same axis. In an embodiment wherein the tablet core is multilayered, it is important to ensure that the cores are always the right way up along the A-B axis. A suitable detection device arranged in cooperation with a press coater can read whether the cores are in the correct position entering the press coater die, and reject those that are not. Thus, providing a means of in-process control.

Using a colourant such as ferric oxide or excipients opaque to x-rays in a core containing only a single layer can also be advantageous to ensure that a core is correctly positioned with a coating. As an additional in-process control is achieved by means of a light or radiation detector suitably positioned in relation to the press-coater to inspect finished tablets to ensure that for a given dosage form, its core is correctly positioned within its coating.

During the compression of the coating around the core, the coating material above and below the core (the material along and about the (A-B) axis) is relatively highly compacted and dense. On the other hand, the coating material disposed along and about the (X-Y) axis is subjected to lower compaction forces and is relatively less dense. Accordingly, the material about the (X-Y) axis is relatively porous and permissive towards the ingress of aqueous media. Because of the slightly less dense nature of the coating material along this axis, and because the formulator has the latitude to influence the coating thickness, the rate of ingress of the aqueous medium through the coating along the direction of the X-Y axis can be closely controlled. Once an aqueous medium contacts the core, the core may react by swelling and/or gelling or effervescing thereby to break open the core generally along the direction of ingress of the aqueous media (i.e. the X-Y axis) to form to essentially two hemispheres of coating material that may remain conjoined. In this opened form, the dosage form may have the appearance of an opened shell. The reaction of the core material to the presence of the aqueous medium is likewise in part responsible for controlling the release of drug substance from the core.

The hardness of the dosage form is preferably at least 60 Newtons, e.g. 60 to 80 Newtons, and more particularly 60 to 75 Newtons. Hardness may be measured according to a process described in The European Pharmacopoeia 4, 2.9.8 at page 201. The test employs apparatus consisting of 2 opposing jaws, one of which moves towards the other. The flat surfaces of the jaws are perpendicular to the direction of movement. The crushing surfaces of the jaws are flat and larger than the zone of contact with the dosage form. The apparatus is calibrated using a system with a precision of one Newton. The dosage form is placed between the jaws. For each measurement, the dosage form is oriented in the same way with respect to the direction of the applied force. Measurements are carried out on 10 tablets. Results are expressed in terms of the mean, minimum and maximum values (in Newtons) of the force needed to crush the dosage form.

Dosage forms having a hardness within this range are mechanically robust to withstand forces generated in the stomach, particularly in the presence of food. Furthermore, the dosage forms are sufficiently porous about the (X-Y) plane of the tablet to permit ingress of physiological media to the core at an appropriate rate to ensure lag times referred to herein above.

The invention provides in another aspect, a method of forming press-coated dosage forms as herein above described. They may be formed on conventional press coating equipment. Typically such equipment is composed of a series of die are arranged on a rotating platform. The die are removably mounted in the platform such that differently sized die may be employed as appropriate. Each die is hollow to receive a lower punch. The punch is positioned within the die such that the upper surface of the punch and the inner surface of the die define a volume for receiving a precise amount coating material. Once loaded, the platform is rotated until the die is positioned under an upper punch. The upper punch is then urged down onto the coating material under a defined compression force and the coating material is precompressed or tamped between the upper and lower punch. A pre-formed core is then fed into die to rest on the tamped coating. Conventional press coating apparatus may be equipped with centering devices that enable cores to be positioned both vertically and radially. This might be achieved by a tamping process, whereby an initial amount of coating material is placed in a die and is tamped with a shaped punch, such as a pin punch, that leaves an indentation in the coating material in which to receive a core. Thereafter, in a second filling operation, a precise amount of coating material is fed into the die to cover the core, and an upper punch compresses the coating material with a defined compaction force to form press-coated dosage forms.

The compression force applied during the tamping process is relatively light and is just sufficient to provide a bed of coating material to receive the core and to prevent movement of the coating material as a result of centrifugal force. Subsequent compression to form the dosage form may be adjusted to give a requisite hardness.

Preferably, this compression force is 400 kg, although this may be adjusted by +/- 30% in order to give tablets of the required hardness.

The amount of coating material fed into the die can be precisely defined having regard to the density of the coating material to ensure after compression that the dosage form is formed with the required coating thickness about the (A-B) axis; and the dimensions of the die is selected to provide the thickness about the X-Y axis. Should it be necessary to change the thickness of the coating, die of appropriate internal dimensions may be placed in the rotating platform, and the amount of coating material fed into the die may be adjusted accordingly.

Suitable rotary tablet machines having high process speeds are known in the art and need no further discussion here.

Cores may likewise be formed using a conventional rotary tablet machine. Cores are preferably compressed under compression forces sufficient to provide cores having a hardness of about 60 Newtons at least, e.g. 50 to 70 Newtons. Cores having hardness in this range give desired release characteristics. If desired, the cores can be formed at the same time as the press coated tablets are produced. In such case, one might employ a Manesty Dry Cota. Such a press consists of two side-by-side and inter-connected presses where the core is made on one press before being mechanically transferred to the other press for compression coating. Such equipment and techniques for making dosage forms using such equipment are known in the art and no more needs to be said about this here.

Cores are preferably formed according to wet granulation techniques generally known in the art. In a typical procedure, core materials are sieved and blended. Granulating fluid, typically water is then added to the blend and the mixture is homogenized to form a granulate, which is then sprayed dried or dried on a fluid bed drier to obtain a granulate with requisite residual moisture. Preferably the residual moisture content is from about 0.4 to 2.0% by weight. The granulate is then sized by passing it through screens of desired aperture. At this stage, any adjuvants are sized and added to the granulate to form the core composition suitable for compression. The skilled person will appreciate that a coating composition can be formed in an analogous manner.

The skilled person will also appreciate that granulates may be obtained having a range of particle sizes. It is preferred that the coating granulate has a fine fraction that is less than 30%. By "fine fraction" is meant granulate having particle size of up to about 63 microns.

There now follows a series of examples that serve to illustrate the invention.

### Example 1

A core containing drug substance is prepared for the press coated system as follows. The composition of the core is detailed in Table 1. Lactose monohydrate (Lactose Pulvis·H₂O^{®}, Danone, France and Lactose Fast Flo^{®} NF 316, Foremost Ing. Group, USA) is a filling agent with interesting technical and functional properties. Lactose Pulvis·H₂O is used in a blend prepared by wet granulation and Lactose Fast Flo is used in a blend prepared for direct compression. Microcrystalline cellulose (Avicel^{®} pH 101, FMC International, Ireland) is used as an insoluble diluent for direct compression. Polyvinyl pyrrolidone (Plasdone^{®} K29-32, ISP Technology, USA) is a granulating agent, soluble in water, which has the ability of binding the powder particles. Croscarmellose sodium (Ac-Di-Sol^{®}, FMC Corporation, USA) is used in the formulation as a super disintegrant. As the external phase, magnesium stearate (Merck, Switzerland) was added as a lubricant and silicon dioxide (Aerosil^{®} 200, Degussa AG, Germany) in order to improve flow properties of the granular powder.

**Table 1:**

| **Ingredients** | **Content (mg/tablet)** |
|---|---|
| Drug Substance A | 5.00 |
| Lactose (Lactose Pulvis H₂O NF 316) | 39.10 |
| Polyvinyl pyrrolidone (Plasdone^{®} K29-32) | 4.00 |
| Sodium carboxymethyl cellulose (Ac-Di-Sol^{®}) | 11.00 |
| Magnesium stearate | 0.60 |
| Silicon dioxide (Aerosil^{®} 200) | 0.30 |
| Total | 60.00 |

The coating material is of a hydrophobic, water insoluble nature. This coating is composed of dibasic calcium phosphate (Emcompress^{®}, Mendell, USA) and glyceryl behenate (Compritol^{®} 888ATO, Gattefossé, France). Polyvinylpyrrolidone (Plasdone^{®} K29-32) is a granulating agent, soluble in water, which has the ability of binding the powder particles. Yellow ferric oxide (Sicovit^{®} Yellow 10, BASF, Germany) was added as a dye. A detailed composition of this barrier blend is given in table 2.

**Table 2:**

| Composition of the coating | |
|---|---|
| **Ingredients** | **Content (%)** |
| Dibasic calcium phosphate (Emcompress^{®}) | 50.00 |
| Glyceryl Behenate (Compritol^{®} 888 ATO) | 40.00 |
| Polyvinylpyrrolidone (Plasdone^{®} K29-32) | 8.40 |
| Yellow Ferric Oxide (Sicovit^{®} yellow 10 E 172) | 0.10 |
| Silicon dioxide (Aerosil^{®} 200) | 0.50 |
| Magnesium stearate | 1.00 |
| Total | 100.00 |

The required amounts of drug substance A, Ac-Di-Sol^{®}, Lactose Pulvis H₂O^{®}, Plasdone^{®} K29-32 were weighed and manually sieved with a screen having 0.710 mm apertures. The components were homogeneously mixed in a Niro-Fielder PMA 25-litre mixing granulator for 6 min at impeller speed 250 rpm without chopper. Subsequently, the granulating solution (purified water, 25.47 % of the weight of the dry blend) was added within 4 min at impeller speed 250 rpm and chopper speed 1500 rpm, using a nozzle H1/4VV-95015 (spraying rate of 250 g/min). Mixing was continued for homogenisation and massing of the wet mass for 3 min at impeller speed 500 rpm and chopper speed 3000 rpm.

The mixed wet granulate is then dried in a Glatt WSG5 fluidised air bed drier. The inlet temperature is maintained at 45°C during drying. The drying lasted 20 min to obtain a granulate with a residual moisture less than 2.5%. The yielded dry granulate is calibrated in a Frewitt MGI 205 granulator using a screen with 0.8 mm apertures for 3 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 25-litre mixing granulator, followed by Aerosil^{®} 200 and then by the other half of the dry granulate. The ingredients are mixed for 2 min at impeller speed 250 rpm. Finally, magnesium stearate is added and mixing is continued for 2 min at impeller speed 250 rpm.

The coating blend is prepared according to the process described below. Batch size for the barrier blend is 13 kg. Weighed amounts of Emcompress^{®}, Compritol^{®} 888 ATO, Lactose pulvis·H2O^{®}, Plasdone^{®} K29-32 and Sicovit^{®} Yellow 10 E 172 are manually sieved with a screen having 0.710 mm apertures. They are placed in a Niro-Fielder PMA 65-litre mixing granulator. Then, the components are homogeneously mixed for 6 min, at impeller speed 200 rpm, without chopper. Subsequently, the granulating solution (purified water, 8.12 % of the weight of the dry blend) is added within 2 min at impeller speed 200 rpm and chopper speed 1500 rpm using a nozzle 4,9 (spraying rate of 520 g/min). Mixing is continued for homogenisation and massing for 1 min at impeller speed 400 rpm and chopper speed 3000 rpm.

The mixed wet granulate is then dried in a Niro-Fielder TSG 2 fluidised air bed dryer. The inlet temperature is maintained at 45°C during drying. The drying lasted 33 min to have residual moisture less than 2.5%. The yielded dry granulate is calibrated in a Frewitt MGI 205 granulator using a screen having 0.8 mm apertures for 4 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 65-litre, followed by Aerosil^{®} 200 and then by the other half of the dry granulate. The ingredients are mixed for 2 min at impeller speed 200 rpm, without chopper. Finally, magnesium stearate is added and mixing is continued for 2 more minutes at impeller speed 200 rpm, without chopper.

440 mg of coating blend is press coated on a core to provide press coated tablets (9 mm diameter). 305 mg of coating blend is press coated on a core to provide press coated tablets (8 mm diameter). These different press coatings are made utilising a Kilian RUD tabletting machine. First and second loading hoppers are filled up with the coating granulate. Between the two loading hoppers, the machine is equipped with a transfer system adapted to feed the cores. For each tablet, the first loading hopper supplies with about half of the quantity to be applied to the core. Then, the feeding system provides and positions a core centred in the die. Subsequently, the second loading hopper supplies with the other half of the quantity to be applied to the core. The compression step then occurs.

### Example 2

The in vitro dissolution profile of a tablet containing a 5mg loading of drug substance A prepared according to the method of Example 1 is determined using USP dissolution apparatus No. 2 (paddles) and stationary baskets and applying a stirring rate of 100 rpm. The dissolution medium was purified water, with a volume of 1000 ml.

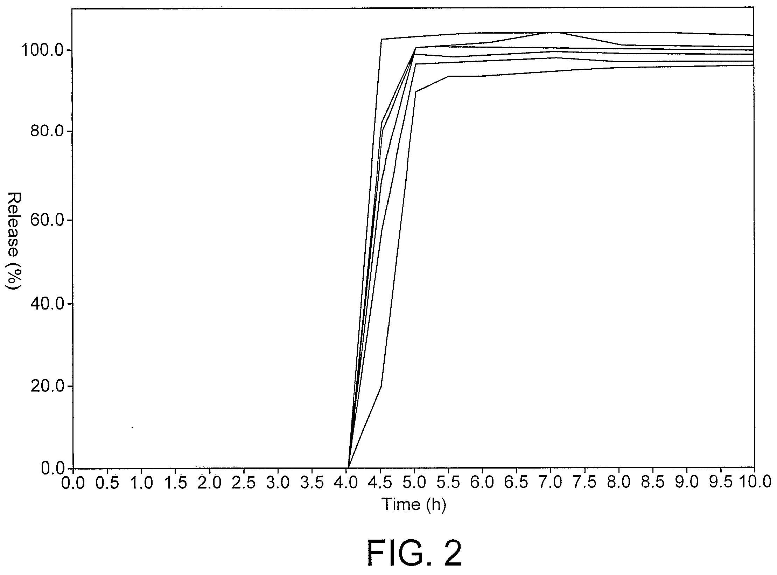

Figure 2 shows the release profiles of several tablets formed according to the above formulation and methodology. The figure clearly shows that it is possible to obtain lag times with a very high degree of precision.

### Example 3 : Formulation 53Q1 (1 hour time lag, 4 hour sustained release)

A core containing drug substance is prepared for the press coated system as follows. The composition of the core is detailed in Table 3. Lactose monohydrate (Lactose Pulvis·H₂O^{®}, Danone, France and Lactose Fast Flo^{®} NF 316, Foremost Ing. Group, USA) is a filling agent with interesting technical and functional properties. Lactose Pulvis·H₂O is used in a blend prepared by wet granulation and Lactose Fast Flo is used in a blend prepared for direct compression. Hydroxypropylmethyl cellulose (Methocel K4M) is used to modify the release of the active agent (Zaleplon). Polyvinyl pyrrolidone (Plasdone^{®} K29-32, ISP Technology, USA) is a granulating agent, soluble in water, which has the ability of binding the powder particles. Sodium lauryl sulphate is a surfactant which helps to wet or hydrate the core and may help to solubilise the active agent. Red ferric oxide is added as a visual indicator to assist in ensuring that the core is correctly centered in the tablet punch. As the external phase, magnesium stearate (Merck, Switzerland) was added as a lubricant and silicon dioxide (Aerosil^{®} 200, Degussa AG, Germany) in order to improve flow properties of the granular powder.

**Table 3: Formulation of the core 1041/32E1 made with 1041/21SR1**

| **Ingredients** | **Content (mg/tablet)** | **%** |
|---|---|---|
| Zaleplon | 15.00 | 25.00 |
| Lactose (Lactose Pulvis H₂O NF 316) | 11.00 | 18.33 |
| Polyvinyl pyrrolidone (Plasdone^{®} K29-32) | 3.00 | 5.00 |
| Methocel K4M (hydroxypropylmethyl cellulose) | 22.00 | 36.67 |
| Magnesium stearate | 1.00 | 1.67 |
| Silicon dioxide (Aerosil^{®} 200) | 0.60 | 1.00 |
| Sodium lauryl sulphate | 7.00 | 11.67 |
| Red ferric oxide | 0.40 | 0.67 |
| Total | 60.00 | 100.00 |

The coating material is of a hydrophobic, water insoluble nature. This coating is composed of dibasic calcium phosphate dihydrate (Calipharm^{®}, CAS 7789-77-7) and glyceryl behenate (Compritol^{®} 888ATO, Gattefossé, France). Polyvinylpyrrolidone (Plasdone^{®} K29-32) is a granulating agent, soluble in water, which has the ability of binding the powder particles. Yellow ferric oxide (Sicovit^{®} Yellow 10, BASF, Germany) was added as a dye. Xylitol 300 (Xylisorb, CAS 87-99-0) is used as a hydrophilic compound, whilst sodium lauryl sulphate (CAS 151-21-3) is added as a hydrophilic compound and solubilising agent.

A detailed composition of this barrier blend is given in table 4.

**Table 4: Composition of the coating**

| **Ingredients** | **mg/tab** | **Content (%)** |
|---|---|---|
| Dibasic calcium phosphate dihydrate (Calipharm^{®}, CAS 7789-77-7) | 145.75 | 32.75 |
| Glyceryl Behenate (Compritol^{®} 888 ATO) | 116.60 | 26.20 |
| Xylitol 300 (Xylisorb, CAS 87-99-0) | 133.50 | 30.00 |
| Sodium lauryl sulphate (CAS 151-21-3) | 20.00 | 4.49 |
| Polyvinylpyrrolidone (Plasdone^{®} K29-32) | 24.49 | 5.50 |
| Yellow Ferric Oxide (Sicovit^{®} yellow 10 E 172) | 0.29 | 0.07 |
| Silicon dioxide (Aerosil^{®} 200) | 1.46 | 0.33 |
| Magnesium stearate | 2.92 | 0.66 |
| Total | 445.00 | 100.00 |

The required amounts of Zaleplon, Methocel K4M, Lactose Pulvis H₂O^{®}, Plasdone^{®} K29-32 were weighed and manually sieved with a screen having 0.710 mm apertures. The components were homogeneously mixed in a Niro-Fielder PMA 25-litre mixing granulator for 6 min at impeller speed 250 rpm without chopper. Subsequently, the granulating solution (purified water, 25.47 % of the weight of the dry blend) was added within 4 min at impeller speed 250 rpm and chopper speed 1500 rpm, using a nozzle H1/4VV-95015 (spraying rate of 250 g/min). Mixing was continued for homogenisation and massing of the wet mass for 3 min at impeller speed 500 rpm and chopper speed 3000 rpm.

The mixed wet granulate is then dried in a Glatt WSG5 fluidised air bed drier. The inlet temperature is maintained at 45°C during drying. The drying lasted 20 min to obtain a granulate with a residual moisture less than 2.5%. The yielded dry granulate is calibrated in a Frewitt MGI 205 granulator using a screen with 0.8 mm apertures for 3 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 25-litre mixing granulator, followed by Aerosil^{®} 200 and then by the other half of the dry granulate. The ingredients are mixed for 2 min at impeller speed 250 rpm. Finally, magnesium stearate is added and mixing is continued for 2 min at impeller speed 250 rpm.

The coating blend is prepared according to the process described below. Batch size for the barrier blend is 13 kg. Weighed amounts of Calipharm^{®}, Compritol^{®} 888 ATO, Lactose pulvis·H2O^{®}, Plasdone^{®} K29-32 and Sicovit^{®} Yellow 10 E 172 are manually sieved with a screen having 0.710 mm apertures. They are placed in a Niro-Fielder PMA 65-litre mixing granulator. Then, the components are homogeneously mixed for 6 min, at impeller speed 200 rpm, without chopper. Subsequently, the granulating solution (purified water, 8.12 % of the weight of the dry blend) is added within 2 min at impeller speed 200 rpm and chopper speed 1500 rpm using a nozzle 4,9 (spraying rate of 520 g/min). Mixing is continued for homogenisation and massing for 1 min at impeller speed 400 rpm and chopper speed 3000 rpm.

The mixed wet granulate is then dried in a Niro-Fielder TSG 2 fluidised air bed dryer. The inlet temperature is maintained at 45°C during drying. The drying lasted 33 min to have residual moisture less than 2.5%. The yielded dry granulate is calibrated in a Frewitt MGI 205 granulator using a screen having 0.8 mm apertures for 4 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 65-litre, followed by Aerosil^{®} 200 and then by the other half of the dry granulate. The ingredients are mixed for 2 min at impeller speed 200 rpm, without chopper. Finally, magnesium stearate is added and mixing is continued for 2 more minutes at impeller speed 200 rpm, without chopper.

440 mg of coating blend is press coated on a core to provide press coated tablets (9 mm diameter). 305 mg of coating blend is press coated on a core to provide press coated tablets (8 mm diameter). These different press coatings are made utilising a Kilian RUD tabletting machine. First and second loading hoppers are filled up with the coating granulate. Between the two loading hoppers, the machine is equipped with a transfer system adapted to feed the cores. For each tablet, the first loading hopper supplies with about half of the quantity to be applied to the core. Then, the feeding system provides and positions a core centred in the die. Subsequently, the second loading hopper supplies with the other half of the quantity to be applied to the core. The compression step then occurs.

### Example 4 : Formulation 51Q1 (2 hour time lag, immediate release)

A core containing drug substance is prepared for the press coated system as follows. The composition of the core is detailed in Table 5. Lactose monohydrate (Lactose Pulvis·H₂O^{®}, Danone, France and Lactose Fast Flo^{®} NF 316, Foremost Ing. Group, USA) is a filling agent with interesting technical and functional properties. Lactose Pulvis·H₂O is used in a blend prepared by wet granulation and Lactose Fast Flo is used in a blend prepared for direct compression. Croscarmellose sodium (Ac-Di-Sol, FMC Corporation, USA) is used in the formulation as a super disintegrant. Polyvinyl pyrrolidone (Plasdone^{®} K29-32, ISP Technology, USA) is a granulating agent, soluble in water, which has the ability of binding the powder particles. Sodium lauryl sulphate is a surfactant which helps to wet or hydrate the core and may help to solubilise the active agent. Red ferric oxide is added as a visual indicator to assist in ensuring that the core is correctly centered in the tablet punch. As the external phase, magnesium stearate (Merck, Switzerland) was added as a lubricant and silicon dioxide (Aerosil^{®} 200, Degussa AG, Germany) in order to improve flow properties of the granular powder.

**Table 5: Formulation of the core 1041/29E1 made with 1041/02FR1**

| **Ingredients** | **Content (mg/tablet)** | **%** |
|---|---|---|
| Zaleplon | 15.00 | 25.00 |
| Lactose (Lactose Pulvis H₂O NF 316) | 25.80 | 43.00 |
| Polyvinyl pyrrolidone (Plasdone^{®} K29-32) | 4.00 | 6.67 |
| Sodium carboxymethyl cellulose (Ac-Di-Sol^{®}) | 11.00 | 18.33 |
| Magnesium stearate | 0.60 | 1.00 |
| Silicon dioxide (Aerosil^{®} 200) | 0.30 | 0.50 |
| Sodium lauryl sulphate | 3.00 | 5.00 |
| Red ferric oxide | 0.30 | 0.50 |
| Total | 60.00 | 100.00 |

The coating material is of a hydrophobic, water insoluble nature. This coating is composed of dibasic calcium phosphate dihydrate (Calipharm^{®}, CAS 7789-77-7) and glyceryl behenate (Compritol^{®} 888ATO, Gattefossé, France). Polyvinylpyrrolidone (Plasdone^{®} K29-32) is a granulating agent, soluble in water, which has the ability of binding the powder particles. Yellow ferric oxide (Sicovit^{®} Yellow 1O, BASF, Germany) was added as a dye. Xylitol 300 (Xylisorb, CAS 87-99-0) is used as a hydrophilic compound, whilst sodium lauryl sulphate (CAS 151-21-3) is added as a hydrophilic compound and solubilising agent.

A detailed composition of this barrier blend is given in table 6.

**Table 6: Composition of the coating**

| **Ingredients** | **mg/tab** | **Content (%)** |
|---|---|---|
| Dibasic calcium phosphate dihydrate (Calipharm^{®}, CAS 7789-77-7) | 173.00 | 38.88 |
| Glyceryl Behenate (Compritol^{®} 888 ATO) | 138.40 | 31.10 |
| Xylitol 300 (Xylisorb, CAS 87-99-0) | 89.00 | 20.00 |
| Sodium lauryl sulphate (CAS 151-21-3) | 10.00 | 2.25 |
| Polyvinylpyrrolidone (Plasdone^{®} K29-32) | 29.06 | 6.53 |
| Yellow Ferric Oxide (Sicovit^{®} yellow 10 E 172) | 0.35 | 0.08 |
| Silicon dioxide (Aerosil^{®} 200) | 1.73 | 0.39 |
| Magnesium stearate | 3.46 | 0.78 |
| Total | 445.00 | 100.00 |

The required amounts of Zaleplon, Methocel K4M, Lactose Pulvis H₂O^{®}, Plasdone^{®} K29-32 were weighed and manually sieved with a screen having 0.710 mm apertures. The components were homogeneously mixed in a Niro-Fielder PMA 25-litre mixing granulator for 6 min at impeller speed 250 rpm without chopper. Subsequently, the granulating solution (purified water, 25.47 % of the weight of the dry blend) was added within 4 min at impeller speed 250 rpm and chopper speed 1500 rpm, using a nozzle H1/4VV-95015 (spraying rate of 250 g/min). Mixing was continued for homogenisation and massing of the wet mass for 3 min at impeller speed 500 rpm and chopper speed 3000 rpm.

The mixed wet granulate is then dried in a Glatt WSG5 fluidised air bed drier. The inlet temperature is maintained at 45°C during drying. The drying lasted 20 min to obtain a granulate with a residual moisture less than 2.5%. The yielded dry granulate is calibrated in a Frewitt MGI 205 granulator using a screen with 0.8 mm apertures for 3 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 25-litre mixing granulator, followed by Aerosil^{®} 200 and then by the other half of the dry granulate. The ingredients are mixed for 2 min at impeller speed 250 rpm. Finally, magnesium stearate is added and mixing is continued for 2 min at impeller speed 250 rpm.

The coating blend is prepared according to the process described below. Batch size for the barrier blend is 13 kg. Weighed amounts of Calipharm^{®}, Compritol^{®} 888 ATO, Lactose pulvis·H2O^{®}, Plasdone^{®} K29-32 and Sicovit^{®} Yellow 10 E 172 are manually sieved with a screen having 0.710 mm apertures. They are placed in a Niro-Fielder PMA 65-litre mixing granulator. Then, the components are homogeneously mixed for 6 min, at impeller speed 200 rpm, without chopper. Subsequently, the granulating solution (purified water, 8.12 % of the weight of the dry blend) is added within 2 min at impeller speed 200 rpm and chopper speed 1500 rpm using a nozzle 4,9 (spraying rate of 520 g/min). Mixing is continued for homogenisation and massing for 1 min at impeller speed 400 rpm and chopper speed 3000 rpm.

The mixed wet granulate is then dried in a Niro-Fielder TSG 2 fluidised air bed dryer. The inlet temperature is maintained at 45°C during drying. The drying lasted 33 min to have residual moisture less than 2.5%. The yielded dry granulate is calibrated in a Frewitt MGI 205 granulator using a screen having 0.8 mm apertures for 4 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 65-litre, followed by Aerosil^{®} 200 and then by the other half of the dry granulate. The ingredients are mixed for 2 min at impeller speed 200 rpm, without chopper. Finally, magnesium stearate is added and mixing is continued for 2 more minutes at impeller speed 200 rpm, without chopper.

440 mg of coating blend is press coated on a core to provide press coated tablets (9 mm diameter). 305 mg of coating blend is press coated on a core to provide press coated tablets (8 mm diameter). These different press coatings are made utilising a Kilian RUD tabletting machine. First and second loading hoppers are filled up with the coating granulate. Between the two loading hoppers, the machine is equipped with a transfer system adapted to feed the cores. For each tablet, the first loading hopper supplies with about half of the quantity to be applied to the core. Then, the feeding system provides and positions a core centred in the die. Subsequently, the second loading hopper supplies with the other half of the quantity to be applied to the core. The compression step then occurs.

### Example 5 : Formulation 54Q1 (2 hour time lag, 2 hour sustained release)

A core containing drug substance is prepared for the press coated system as follows. The composition of the core is detailed in Table 7. Lactose monohydrate (Lactose Pulvis-H₂O^{®}, Danone, France and Lactose Fast Flo^{®} NF 316, Foremost Ing. Group, USA) is a filling agent with interesting technical and functional properties. Lactose Pulvis·H₂O is used in a blend prepared by wet granulation and Lactose Fast Flo is used in a blend prepared for direct compression. Hydroxypropylmethyl cellulose (Methocel K100LV) is used to modify the release of the active agent (Zaleplon). Polyvinyl pyrrolidone (Plasdone^{®} K29-32, ISP Technology, USA) is a granulating agent, soluble in water, which has the ability of binding the powder particles. Sodium lauryl sulphate is a surfactant which helps to wet or hydrate the core and may help to solubilise the active agent. Red ferric oxide is added as a visual indicator to assist in ensuring that the core is correctly centered in the tablet punch. As the external phase, magnesium stearate (Merck, Switzerland) was added as a lubricant and silicon dioxide (Aerosil^{®} 200, Degussa AG, Germany) in order to improve flow properties of the granular powder.

**Table 7: Formulation of the core 1041/33E1 made with 1041/22SR1**

| **Ingredients** | **Content (mg/tablet)** | **%** |
|---|---|---|
| Zaleplon | 15.00 | 25.00 |
| Lactose (Lactose Pulvis H₂O NF | 11.00 | 18.33 |
| 316) | | |
| Polyvinyl pyrrolidone (Plasdone^{®} K29-32) | 3.00 | 5.00 |
| Methocel K4M (hydroxypropylmethyl cellulose) | 22.00 | 36.67 |
| Magnesium stearate | 1.00 | 1.67 |
| Silicon dioxide (Aerosil^{®} 200) | 0.60 | 1.00 |
| Sodium lauryl sulphate | 7.00 | 11.67 |
| Red ferric oxide | 0.40 | 0.67 |
| Total | 60.00 | 100.00 |

The coating material is of a hydrophobic, water insoluble nature. This coating is composed of dibasic calcium phosphate dihydrate (Calipharm^{®}, CAS 7789-77-7) and glyceryl behenate (Compritol^{®} 888ATO, Gattefossé, France). Polyvinylpyrrolidone (Plasdone^{®} K29-32) is a granulating agent, soluble in water, which has the ability of binding the powder particles. Yellow ferric oxide (Sicovit^{®} Yellow 10, BASF, Germany) was added as a dye. Xylitol 300 (Xylisorb, CAS 87-99-0) is used as a hydrophilic compound, whilst sodium lauryl sulphate (CAS 151-21-3) is added as a hydrophilic compound and solubilising agent.

A detailed composition of this barrier blend is given in table 8.

**Table 8: Composition of the coating**

| **Ingredients** | **mg/tab** | **Content (%)** |
|---|---|---|
| Dibasic calcium phosphate dihydrate (Calipharm^{®}, CAS 7789-77-7) | 173.00 | 38.88 |
| Glyceryl Behenate (Compritol^{®} 888 ATO) | 138.40 | 31.10 |
| Xylitol 300 (Xylisorb, CAS 87-99-0) | 89.00 | 20.00 |
| Sodium lauryl sulphate (CAS 151-21-3) | 10.00 | 2.25 |
| Polyvinylpyrrolidone (Plasdone^{®} K29-32) | 29.06 | 6.53 |
| Yellow Ferric Oxide (Sicovit^{®} yellow 10 E | 0.35 | 0.08 |
| 172) | | |
| Silicon dioxide (Aerosil^{®} 200) | 1.73 | 0.39 |
| Magnesium stearate | 3.46 | 0.78 |
| Total | 445.00 | 100.00 |

The required amounts of Zaleplon, Methocel K4M, Lactose Pulvis H₂O^{®}, Plasdone^{®} K29-32 were weighed and manually sieved with a screen having 0.710 mm apertures. The components were homogeneously mixed in a Niro-Fielder PMA 25-litre mixing granulator for 6 min at impeller speed 250 rpm without chopper. Subsequently, the granulating solution (purified water, 25.47 % of the weight of the dry blend) was added within 4 min at impeller speed 250 rpm and chopper speed 1500 rpm, using a nozzle H1/4W-95015 (spraying rate of 250 g/min). Mixing was continued for homogenisation and massing of the wet mass for 3 min at impeller speed 500 rpm and chopper speed 3000 rpm.

The mixed wet granulate is then dried in a Glatt WSG5 fluidised air bed drier. The inlet temperature is maintained at 45°C during drying. The drying lasted 20 min to obtain a granulate with a residual moisture less than 2.5%. The yielded dry granulate is calibrated in a Frewitt MGI 205 granulator using a screen with 0.8 mm apertures for 3 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 25-litre mixing granulator, followed by Aerosil^{®} 200 and then by the other half of the dry granulate. The ingredients are mixed for 2 min at impeller speed 250 rpm. Finally, magnesium stearate is added and mixing is continued for 2 min at impeller speed 250 rpm.

The coating blend is prepared according to the process described below. Batch size for the barrier blend is 13 kg. Weighed amounts of Calipharm^{®}, Compritol^{®} 888 ATO, Lactose pulvis·H2O^{®}, Plasdone^{®} K29-32 and Sicovit^{®} Yellow 10 E 172 are manually sieved with a screen having 0.710 mm apertures. They are placed in a Niro-Fielder PMA 65-litre mixing granulator. Then, the components are homogeneously mixed for 6 min, at impeller speed 200 rpm, without chopper. Subsequently, the granulating solution (purified water, 8.12 % of the weight of the dry blend) is added within 2 min at impeller speed 200 rpm and chopper speed 1500 rpm using a nozzle 4,9 (spraying rate of 520 g/min). Mixing is continued for homogenisation and massing for 1 min at impeller speed 400 rpm and chopper speed 3000 rpm.

The mixed wet granulate is then dried in a Niro-Fielder TSG 2 fluidised air bed dryer. The inlet temperature is maintained at 45°C during drying. The drying lasted 33 min to have residual moisture less than 2.5%. The yielded dry granulate is calibrated in a Frewitt MGI 205 granulator using a screen having 0.8 mm apertures for 4 min at speed 244 osc/min (graduation 7). Appropriate amounts of Aerosil^{®} 200 and magnesium stearate are manually sieved using a screen with 1.0 mm apertures. Half of the dry granulate is put in a Niro-Fielder PMA 65-litre, followed by Aerosil^{®} 200 and then by the other half of the dry granulate. The ingredients are mixed for 2 min at impeller speed 200 rpm, without chopper. Finally, magnesium stearate is added and mixing is continued for 2 more minutes at impeller speed 200 rpm, without chopper.

440 mg of coating blend is press coated on a core to provide press coated tablets (9 mm diameter). 305 mg of coating blend is press coated on a core to provide press coated tablets (8 mm diameter). These different press coatings are made utilising a Kilian RUD tabletting machine. First and second loading hoppers are filled up with the coating granulate. Between the two loading hoppers, the machine is equipped with a transfer system adapted to feed the cores. For each tablet, the first loading hopper supplies with about half of the quantity to be applied to the core. Then, the feeding system provides and positions a core centred in the die. Subsequently, the second loading hopper supplies with the other half of the quantity to be applied to the core. The compression step then occurs.

### Example 6

The *in vitro* dissolution profile of tablets each containing a 5mg loading of Zaleplon prepared according to the method of Examples 3, 4 and 5 respectively is determined using USP dissolution apparatus No. 2 (paddles) and stationary baskets and applying a stirring rate of 100 rpm. The dissolution medium was 0.02% sodium lauryl sulphate in 500ml distilled water, with a volume of 1000 ml.

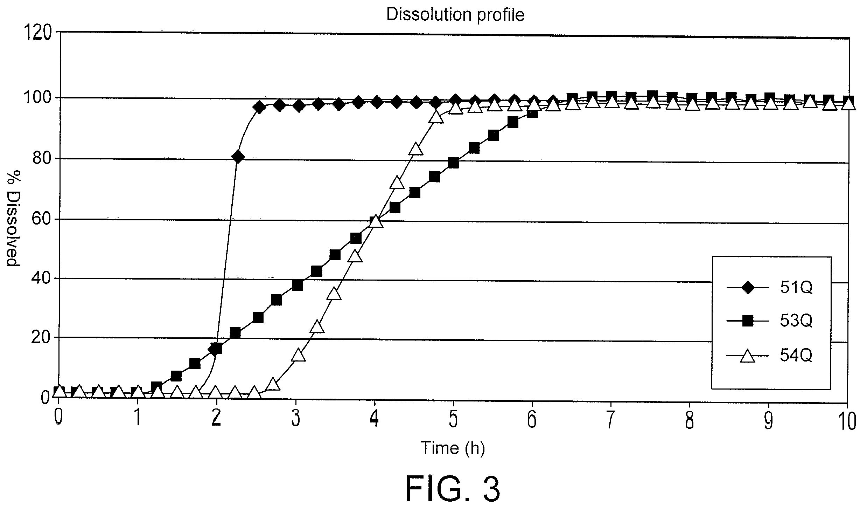

Figure 3 illustrates the release of Zaleplon from the formulations of Examples 3-5. A lag time of at least one hour is observed in each case, followed by immediate release (Example 4) or delayed release (Examples 3 and 5) of the active agent.

## Claims

1. A dosage form containing a drug substance useful in the treatment of insomnia,
said dosage form comprising a layer or coating surrounding a drug substance containing core matrix;
said dosage form being adapted to release said drug substance after a lag time during which substantially no drug substance is released, said lag time being from 1 to 4 hours after administration of the dosage form;
wherein the coating comprises less than 10% of swellable or gellable materials;
wherein the coating comprises one or more insoluble or poorly water soluble hydrophobic materials selected from fatty acids or their esters or salts; long chain fatty alcohols; polyoxyethylene alkyl ethers; polyoxyethylene stearates; sugar esters; lauroyl macrogol-32 glyceryl, stearoyl macrogol-32 glyceryl, waxy substances, calcium phosphate salts and combinations thereof; and
wherein the drug substance is zaleplon.

2. A dosage form according to claim 1, wherein the release of said drug substance from the dosage form is pH-independent.

3. A dosage form according to claim 1 or claim 2, which is provided as a unit (single component) dose.

4. A dosage form according to any preceding claim, wherein said dosage form is adapted to obtain a controlled release of said drug substance in vitro when measured by the USP Paddle Dosage form (type II apparatus) at 100 rpm in 1000ml of an aqueous medium, such that during said lag time not more than 10% of said drug substance is released.

5. A dosage form as claimed in claim 4, wherein said dosage form is adapted to obtain a controlled release of said drug substance in vitro when measured by the USP Paddle Method (type II apparatus) at 100 rpm in 1000ml of an aqueous medium, such that during said lag time not more than 10% of drug substance is released, at least about 25 to 60% is released within 5 hours, and at least about 80% is released after 7 hours.

6. A dosage form according to claim 4 or claim 5, wherein said aqueous medium comprises (a) 0.1M HCl and phosphate buffer (pH 6.8) or (b) 0.02% sodium lauryl sulphate in 500mls distilled water or (c) purified water, and wherein said measurement is taken at 37°C.

7. A dosage form according to any preceding claim, wherein the drug substance is present in an amount of 5 to 50mg per dosage form.

8. A dosage form according to any preceding claim, wherein said dosage form contains one or more drug substances and a release controlling agent.

9. A dosage form according to claim 8, wherein the release controlling agent is provided in a matrix in which said drug substance is dissolved or dispersed.

10. A dosage form according to any preceding claim, wherein the one or more insoluble or poorly water soluble hydrophobic materials are selected from calcium phosphate salts and glyceryl behenate.

11. A dosage form according to any preceding claim, wherein said dosage form is in the form of a press-coated tablet.

12. A dosage form according to any preceding claim, which is suitable for treating sleep latency and/or wakening events.

13. A dosage form according to any one of claims 1 to 12 for use in the treatment of insomnia.

## Patentansprüche

1. Dosierungsform, die einen zur Behandlung von Schlafstörungen geeigneten Wirkstoff enthält, wobei
die Dosierungsform eine Schicht oder einen Überzug umfasst, die bzw. der eine Wirkstoff enthaltende Kernmatrix umgibt;
die Dosierungsform ausgelegt ist, um den Wirkstoff nach einer Verzögerung freizusetzen, während der im Wesentlichen kein Wirkstoff freigesetzt wird, wobei die Verzögerung 1 bis 4 Stunden nach Verabreichung der Dosierungsform beträgt;
wobei der Überzug weniger als 10% quellbare oder gelierbare Materialien umfasst;
wobei der Überzug ein oder mehr unlösliche oder schlecht wasserlösliche hydrophobe Materialien umfasst, die ausgewählt sind aus Fettsäuren oder deren Estern oder Salzen; langkettigen Fettalkoholen; Polyoxyethylenalkylethern; Polyoxyethylenstearaten; Zuckerestern; Lauroyl-Macrogol-32-Glyceriden, Stearoyl-Macrogol-32-Glyceriden, wachsartigen Stoffen, Calciumphosphatsalzen und Kombinationen davon; und
wobei der Wirkstoff Zaleplon ist.

2. Dosierungsform nach Anspruch 1, wobei die Freisetzung des Wirkstoffs aus der Dosierungsform pH-unabhängig ist.

3. Dosierungsform nach Anspruch 1 oder Anspruch 2, welche als eine Unit-Dose (Einkomponentendosis) bereitgestellt wird.

4. Dosierungsform nach einem der vorhergehenden Ansprüche, wobei die Dosierungsform ausgelegt ist, um bei Messung nach USP Paddle Dosage Form (Typ II-Apparatur) bei 100 UpM in 1000 ml eines wässrigen Mediums in vitro eine derart kontrollierte Freisetzung des Wirkstoffs zu erhalten, dass während der Verzögerung nicht mehr als 10% des Wirkstoffs freigesetzt werden.

5. Dosierungsform nach Anspruch 4, wobei die Dosierungsform ausgelegt ist, um bei Messung nach USP Paddle Dosage Form (Typ II-Apparatur) bei 100 UpM in 1000 ml eines wässrigen Mediums in vitro eine derart kontrollierte Freisetzung des Wirkstoffs zu erhalten, dass während der Verzögerung nicht mehr als 10% des Wirkstoffs freigesetzt werden, innerhalb von 5 Stunden wenigstens etwa 25 bis 60% freigesetzt werden, und nach 7 Stunden wenigstens etwa 80% freigesetzt werden.

6. Dosierungsform nach Anspruch 4 oder Anspruch 5, wobei das wässrige Medium (a) 0,1 M HCl und Phosphatpuffer (pH 6,8) oder (b) 0,02% Natriumlaurylsulfat in 500 ml destilliertem Wasser oder (c) reines Wasser umfasst, und wobei die Messung bei 37°C erfolgt.

7. Dosierungsform nach einem der vorhergehenden Ansprüche, wobei der Wirkstoff in einer Menge von 5 bis 50 mg pro Dosierungsform vorliegt.

8. Dosierungsform nach einem der vorhergehenden Ansprüche, wobei die Dosierungsform ein oder mehr Wirkstoffe und ein die Freisetzung steuerndes Mittel enthält.

9. Dosierungsform nach Anspruch 8, wobei das die Freisetzung steuernde Mittel in einer Matrix bereitgestellt wird, in der der Wirkstoff gelöst oder dispergiert ist.

10. Dosierungsform nach einem der vorhergehenden Ansprüche, wobei die ein oder mehr unlöslichen oder schlecht wasserlöslichen hydrophoben Materialien ausgewählt sind aus Calciumphosphatsalzen und Glycerinbehenat.

11. Dosierungsform nach einem der vorhergehenden Ansprüche, wobei die Dosierungsform in Form einer Manteltablette vorliegt.

12. Dosierungsform nach einem der vorhergehenden Ansprüche, welche zur Behandlung von Schlaflatenz und/oder Aufwachereignissen geeignet ist.

13. Dosierungsform nach einem der Ansprüche 1 bis 12 zur Verwendung bei der Behandlung von Schlafstörungen.

## Revendications

1. Forme posologique contenant une substance médicamenteuse utile dans le traitement de l'insomnie, ladite forme posologique comprenant une couche ou un enrobage entourant une matrice de coeur contenant une substance médicamenteuse ;
ladite forme posologique étant adaptée pour libérer ladite substance médicamenteuse après un temps de latence durant lequel pratiquement aucune substance médicamenteuse n'est libérée, ledit temps de latence étant de 1 à 4 heures après l'administration de la forme posologique ;
dans laquelle l'enrobage comprend moins de 10 % de matériaux gonflables ou gélifiables ;
dans laquelle l'enrobage comprend un ou plusieurs matériau(x) hydrophobe(s) insoluble(s) ou médiocrement soluble(s) dans l'eau choisi(s) parmi les acides gras ou leurs sels ou esters ; les alcools gras à longue chaîne ; les alkyléthers polyoxyéthylénés ; les stéarates polyoxyéthylénés ; les esters de sucre ; le lauroyl-macrogol-32-glycéryle, le stéaroyl-macrogol-32-glycéryle, les substances cireuses, les sels phosphates de calcium et leurs combinaisons ; et
dans laquelle la substance médicamenteuse est le zaléplon.

2. Forme posologique selon la revendication 1, dans laquelle la libération de ladite substance médicamenteuse à partir de la forme posologique est indépendante du pH.

3. Forme posologique selon la revendication 1 ou la revendication 2, qui se présente sous la forme d'une dose unitaire (un seul composant).

4. Forme posologique selon l'une quelconque des revendications précédentes, laquelle forme posologique est adaptée pour réaliser une libération contrôlée de ladite substance médicamenteuse in vitro lors d'une mesure au moyen d'une forme posologique avec agitation par palettes USP (dispositif de type II) à 100 t/min dans 1000 ml d'un milieu aqueux, de sorte que, durant ladite latence, au plus 10 % de ladite substance médicamenteuse soient libérés.

5. Forme posologique selon la revendication 4, laquelle forme posologique est adaptée pour réaliser une libération contrôlée de ladite substance médicamenteuse in vitro lors d'une mesure par le procédé d'agitation par palettes USP (dispositif de type II) à 100 t/min dans 1000 ml d'un milieu aqueux, de sorte que, durant ladite latence, au plus 10 % de ladite substance médicamenteuse soient libérés, au moins environ 25 à 60 % soient libérés en 5 heures, et au moins environ 80 % soient libérés après 7 heures.

6. Forme posologique selon la revendication 4 ou la revendication 5, dans laquelle ledit milieu aqueux comprend (a) du HCl 0,1 M et du tampon phosphate (pH 6,8) ou (b) 0,02 % de laurylsulfate de sodium dans 500 ml d'eau distillée ou (c) de l'eau purifiée, et dans laquelle ladite mesure est effectuée à 37°C.

7. Forme posologique selon l'une quelconque des revendications précédentes, dans laquelle la substance médicamenteuse est présente en une quantité de 5 à 50 mg par forme posologique.

8. Forme posologique selon l'une quelconque des revendications précédentes, laquelle forme posologique contient une ou plusieurs substance(s) médicamenteuse(s) et un agent de contrôle de la libération.

9. Forme posologique selon la revendication 8, dans laquelle l'agent contrôlant la libération est disposé dans une matrice dans laquelle ladite substance médicamenteuse est dissoute ou dispersé.

10. Forme posologique selon l'une quelconque des revendications précédentes, dans laquelle le ou les matériau(x) hydrophobe(s) insoluble(s) ou médiocrement soluble(s) dans l'eau est/sont choisi(s) parmi les sels phosphates de calcium et le béhénate de glycéryle.

11. Forme posologique selon l'une quelconque des revendications précédentes, laquelle forme posologique est sous la forme de comprimés enrobés sous presse.

12. Forme posologique selon l'une quelconque des revendications précédentes, qui convient pour traiter la latence du sommeil et/ou les événements de réveil.

13. Forme posologique selon l'une quelconque des revendications 1 à 12 pour utilisation dans le traitement de l'insomnie.
